# EUROPEAN PATENT APPLICATION

(11) **EP 4 538 368 A1**
(43) Date of publication of application: **16.04.2025**
(21) Application number: 23818806.4
(22) Date of filing: 04.04.2023
(51) Int. Cl.: C12N 9/04, C12N 15/53, C12N 15/81, C12N 1/19, A23K 20/189, C12R 1/84

(54) **THERMAL STABILITY-IMPROVED GLUCOSE OXIDASE GOXM10 MUTANT E361P, DERIVATIVE MUTANT THEREOF, AND USE THEREOF**

(30) Priority: 09.06.2022 CN 202210648703
(71) Applicant: INSTITUTE OF ANIMAL SCIENCE OF CHINESE ACADEMY OF AGRICULTURAL SCIENCES, Beijing 100193 (CN)
(72) Inventor: TU, Tao, Beijing 100193 (CN); HUANG, Huoqing, Beijing 100193 (CN); YAN, Yaru, Beijing 100193 (CN); ZHANG, Wei, Beijing 100193 (CN); LUO, Huiying, Beijing 100193 (CN); YAO, Bin, Beijing 100193 (CN)
(74) Representative: Forresters IP LLP
(86) International application number: PCT/CN2023/086142
(87) International publication number: WO 2023/236638

(57) **Abstract**

The present invention relates to the field of genetic engineering, particularly to glucose oxidase mutant GoxM10 having improved thermal stability, derivative mutant thereof, and application thereof. The present invention researches the stability of glucose oxidase GoxM10 with the high thermal stability, to obtains a mutant with improved stability, thereby promoting the application of Gox in the feed industry.

## Description

### FIELD OF THE INVENTION

The present invention relates to the field of genetic engineering, particularly to glucose oxidase mutant having improved thermal stability, derivative mutant thereof, and application thereof.

### BACKGROUND OF THE INVENTION

Glucose oxidase (Gox; EC1.1.3.4) is an aerobic dehydrogenase with good characteristics which catalyzes the production D-gluconolactone and hydrogen from β-D-glucose using oxygen molecules as electron acceptor under aerobic conditions, wherein D-gluconolactone is subsequently hydrolyzed into gluconic acid and water. In recent years, Gox was concerned widely as a new kind of alternative of the antibiotic and the growth promoter in the feed industry, which is crucial for ensuring animal's health and production performance without harming human health. Numerous applied studies have shown that Gox, as a green feed additive, can be used to prevent gastrointestinal infections and diarrhea in livestock, and has a promoting effect on animal growth.

Currently, most animal feed need to be granulated in high temperature, so the improvement of the thermal stability of Gox is crucial for its widespread application in feed. In addition, it is crucial to maintain high activity in acidic environment in order to maximize its effect in the animal gastrointestinal tract. Therefore, Gox should have good thermal stability and acid stability in order to be applied in the feed industry. The prior art has disclosed the Gox derived from *Aspergillus niger* with the improved stability, such as obtaining the mutant GoxM4 with T₅₀ increased by 7.5°C and the mutant GoxM10 with Tm increased by 9°C compared with that of GoxM4.

### Order of the invention

The order of the present invention is to provide a glucose oxidase mutant GoxM10 with the improved thermal stability.

Another order of the present invention is to provide a gene encoding the above mutant.

Another order of the present invention is to provide a recombinant vector containing the above gene.

Another order of the present invention is to provide a recombinant strain containing the above gene.

Another order of the present invention is to provide application of the above mutant.

Another order of the present invention is to provide a genetic engineering method for preparing the glucose oxidase with the improved thermal stability.

Another order of the present invention is to provide a method of improved the thermal stability of the glucose oxidase.

### SUMMARY OF THE INVENTION

In a preferred embodiment of the present invention, the glucose oxidase with amino acid sequence of SEQ ID NO: 1was performed a site-directed mutagenesis.

In a yet preferred embodiment of the present invention, the amino acid at the site of 203, 219, 338, 361 or 419 of SEQ ID NO: 1 was muted respectively, followed by the double amino acids at the sites of 361/203, 361/219, 361/338, or 361/419/203 of SEQ ID NO: 1 were muted, the three amino acids at the sites of 361/419/203 or 361/419/338 were muted, the four amino acids at the sites of 361/419/193/497 or 361/419/4/471 of SEQ ID NO: 1 were muted, and the six amino acids at the sites of 361/419/4/471/ 193/497 of SEQ ID NO: 1 were muted, in order, to obtain the glucose oxidase mutants with improved acid and thermal stability.

In a further preferred embodiment, the mutation of G203C was performed to the glucose oxidase of the amino acid sequence of SEQ ID NO: 1 to obtain a mutant with improved thermal stability having the amino acid sequence of SEQ ID NO: 2.

In a further preferred embodiment, the gene encoding the mutant of SEQ ID NO: 2 was provided, wherein the said gene has the nucleotide sequence of SEQ ID NO:3.

In a further preferred embodiment, the mutation of E219 was performed to the glucose oxidase of the amino acid sequence of SEQ ID NO: 1 to obtain a mutant with improved thermal stability having the amino acid sequence of SEQ ID NO: 4.

In a further preferred embodiment, the gene encoding the mutant of SEQ ID NO: 4 was provided, wherein the said gene has the nucleotide sequence of SEQ ID NO:5.

In a further preferred embodiment, the mutation of S338P was performed to the glucose oxidase of the amino acid sequence of SEQ ID NO: 1 to obtain a mutant with improved thermal stability having the amino acid sequence of SEQ ID NO: 6.

In a further preferred embodiment, the gene encoding the mutant of SEQ ID NO: 6 was provided, wherein the said gene has the nucleotide sequence of SEQ ID NO:7.

In a further preferred embodiment, the mutation of E361P was performed to the glucose oxidase of the amino acid sequence of SEQ ID NO: 1 to obtain a mutant with improved thermal stability having the amino acid sequence of SEQ ID NO:8.

In a further preferred embodiment, the gene encoding the mutant of SEQ ID NO: 8 was provided, wherein the said gene has the nucleotide sequence of SEQ ID NO:9.

In a further preferred embodiment, the mutation of A419I was performed to the glucose oxidase of the amino acid sequence of SEQ ID NO: 1 to obtain a mutant with improved thermal stability having the amino acid sequence of SEQ ID NO:10.

In a further preferred embodiment, the gene encoding the mutant of SEQ ID NO: 10 was provided, wherein the said gene has the nucleotide sequence of SEQ ID NO:11.

In a further preferred embodiment, the mutation of E361P/G203C was performed to the glucose oxidase of the amino acid sequence of SEQ ID NO: 1 to obtain a mutant with improved thermal stability having the amino acid sequence of SEQ ID NO:12.

In a further preferred embodiment, the gene encoding the mutant of SEQ ID NO: 12 was provided, wherein the said gene has the nucleotide sequence of SEQ ID NO:13.

In a further preferred embodiment, the mutation of E361P/E219P was performed to the glucose oxidase of the amino acid sequence of SEQ ID NO: 1 to obtain a mutant with improved thermal stability having the amino acid sequence of SEQ ID NO:14.

In a further preferred embodiment, the gene encoding the mutant of SEQ ID NO: 14 was provided, wherein the said gene has the nucleotide sequence of SEQ ID NO:15.

In a further preferred embodiment, the mutation of E361P/S338P was performed to the glucose oxidase of the amino acid sequence of SEQ ID NO: 1 to obtain a mutant with improved thermal stability having the amino acid sequence of SEQ ID NO:16.

In a further preferred embodiment, the gene encoding the mutant of SEQ ID NO: 16 was provided, wherein the said gene has the nucleotide sequence of SEQ ID NO:17.

In a further preferred embodiment, the mutation of E361P/A419I was performed to the glucose oxidase of the amino acid sequence of SEQ ID NO: 1 to obtain a mutant with improved thermal stability having the amino acid sequence of SEQ ID NO:18.

In a further preferred embodiment, the gene encoding the mutant of SEQ ID NO: 18 was provided, wherein the said gene has the nucleotide sequence of SEQ ID NO: 19.

In a further preferred embodiment, the mutation of E361P/A419I/G203C was performed to the glucose oxidase of the amino acid sequence of SEQ ID NO: 1 to obtain a mutant with improved thermal stability having the amino acid sequence of SEQ ID NO:20.

In a further preferred embodiment, the gene encoding the mutant of SEQ ID NO: 20 was provided, wherein the said gene has the nucleotide sequence of SEQ ID NO:21.

In a further preferred embodiment, the mutation of E361P/A419I/S338P was performed to the glucose oxidase of the amino acid sequence of SEQ ID NO: 1 to obtain a mutant with improved thermal stability having the amino acid sequence of SEQ ID NO:22.

In a further preferred embodiment, the gene encoding the mutant of SEQ ID NO: 22 was provided, wherein the said gene has the nucleotide sequence of SEQ ID NO:23.

In a further preferred embodiment, the mutation of E361P/A419I/D193N/D497N was performed to the glucose oxidase of the amino acid sequence of SEQ ID NO: 1 to obtain a mutant with improved thermal stability having the amino acid sequence of SEQ ID NO:24.

In a further preferred embodiment, the gene encoding the mutant of SEQ ID NO: 24 was provided, wherein the said gene has the nucleotide sequence of SEQ ID NO:25.

In a further preferred embodiment, the mutation of E361P/A419I/A4D/N471E was performed to the glucose oxidase of the amino acid sequence of SEQ ID NO: 1 to obtain a mutant with improved thermal stability having the amino acid sequence of SEQ ID NO:26.

In a further preferred embodiment, the gene encoding the mutant of SEQ ID NO: 26 was provided, wherein the said gene has the nucleotide sequence of SEQ ID NO:27.

In a further preferred embodiment, the mutation of E361P/A419I/D193N/D497N/A4D/N471E was performed to the glucose oxidase of the amino acid sequence of SEQ ID NO: 1 to obtain a mutant with improved thermal stability having the amino acid sequence of SEQ ID NO:28.

In a further preferred embodiment, the gene encoding the mutant of SEQ ID NO: 28 was provided, wherein the said gene has the nucleotide sequence of SEQ ID NO:29.

The present invention provides recombinant vector respectively comprising the above gene encoding the above mentioned glucose oxidase GOD, wherein the starting vector for the said recombinant expression vector is pPIC9, and the said recombinant expression vector was pPIC9-*goxm10*-G203C,pPIC9-*goxm10*-E219P,pPIC9-*goxm10*-S338P,pPIC9-*goxm10-*E361P,pPIC9-*goxm10*-A419I,pPIC9-*goxm10*-E361P/G203C,pPIC9-*goxm10*-E361P/E 219P,pPIC9-*goxm10*-E361P/S338P,pPIC9-*goxm10*-E361P/A419I,pPIC9-*goxm10*-E36 1P/A419I/G203C,pPIC9-*goxm10*-E361P/A419I/S338P,pPIC9-*goxm10*-E361P/A419I/ D193N/D497N,pPIC9-*goxm10*-E361P/A419I/A4D/N471E or pPIC9-*goxm10*-E361P/A419I/A4D/N471E/D193N/D497N.

The present invention provides a recombinant strain comprising the above gene encoding the each glucose oxidase GOD mutant.

In a further preferred embodiment, the method of preparing glucose oxidase GOD with the improved thermal stability comprises the following steps of transforming the host cells with the recombinant vector containing the gene encoding the above each glucose oxidase GOD mutant to obtain the recombinant strains, culturing the obtained recombinant strains to induce the expression of recombinant glucose oxidase GOD, and recovering and purifying the glucose oxidase GOD.

The present invention also provides the application of the above glucose oxidase GOD mutant with the improved thermal stability, such as the application to the field of the feed or the food.

Compared with the glucose oxidase GoxM10, the each mutant of the present invention has the same optimal reaction temperature of 40°C, and its thermal stability is significantly improved. After being incubated at 65°C for 180 minutes, the remaining enzyme activity of GoxM10 was 61%, and the remaining enzyme activity of the mutant G203C, E219P, S338P, E361P, A419I, E361P/G203C, E361P/E219P, E361P/2338P, E361P/A419I, E361P/A419I/G203C, E361P/A419I/S338P, E361P/A419I/A4D/N471E, E361P/A419I/D193N, and E361P/A419I/A4D/N471E/D193N/D497N is 63%, 69%, 74%, 66%, 75%, 68%, and 76%, respectively. 81%, 72%, 62%, 67%, 75%, and 71%. And, after being incubated at 75°C for 12 minutes, the remaining enzyme activity of the purified enzyme solution of mutant G203C, E219P, S338P, E361P, A419I, E361P/G203C, E361P/E219P, E361P/2338P, E361P/A419I, E361P/A419I/G203C, E361P/A419I/S338P, E361P/A419I/A4D/N471E, E361P/A419I/D193N/D497N and E361P/A419I/A4D/N471E/D193N is 42%, 37%, 42%, 44%, 43%, 47%, 37%, 48%, 50%, 38%, 39%, respectively. At around 42%, 45%, and 44%, and the remaining enzyme activity of of GoxM10 is 36%.

The half-life of GoxM10 at 65°Cis 385 min, while the half-lives of the mutant both E219P and A419I are 433 min being 1.1 times that of GoxM10. The half live of mutants G203C and E361P at 65°Cis 462 min and 495 min, respectively, being 1.2 and 1.3 times that of GoxM10. The half live of mutant S338P are comparable to taht of GoxM10. The half lives of the four double point mutants E361P/G203C, E361P/E219P, E361P/S338P, and E361P/A419I are 495 min, 433 min, 462 min, and 578 min, respectively, being 1.3, 1.1, 1.2, and 1.5 times that of GoxM10 (385 min). The half lives of mutants E361P/A419I/G203C, E361P/A419I/S338P, E361P/A419I/A4D/N471E, E361P/A419I/D193N/D497N, and E361P/A419I/A4D/N471E/D193N/D497N are 433, 450, 495, 553, and 462 min, respectively.

When treated at pH 2.5 for 30 minutes, the relative remaining enzyme activities of mutants E361P/A419I/A4D/N471E, E361P/A419I/D193N/D497N, and E361P/A419I/A4D/N471E/D193N/D497N were 31%, 29%, and 33%, respectively, while the remaining enzyme activity of GoxM10 was 21%. And, when treated at pH 2.75 and 37°C for 60 minutes, the relative remaining enzyme activities of mutants E361P/A419I/A4D/N471E, E361P/A419I/D193N/D497N, and E361P/A419I/A4D/N471E/D193N/D497N were approximately 42%, 46%, and 47%, respectively, while the remaining enzyme activity of GoxM10 was 24%. The optimal pH for mutants E361P/A419I/A4D/N471E, E361P/A419I/D193N/D497N, and E361P/A419I/A4D/N471E/D193N/D497N is consistent with GoxM10 at 6.0.

The improved acid stability or thermal stability of the glucose oxidase mutant provided by the present invention is suitable for application in the fields of food and feed, and has a very broad application prospect.

### BRIEF DESCRIPTIONS OF THE DRAWINGS

Fig. 1 shows the comparison of thermal stability between GoxM10 and the mutants after being treated at 65°C for 180 minutes of treatment ;
Fig.2 shows the comparison of thermal stability between GoxM10 and the mutants after being treated at 75°C for 12 minutest;
Fig.3 shows the comparison of the optimal temperatures between GoxM10 and the mutants;
Fig.4 shows the comparison of acid stability between GoxM10 and mutants after being treated for 30 minutes at pH 2.5;
Fig.5 shows the comparison of acid stability between GoxM10 and mutants after being treated for 60 minutes at pH 2.75;
Fig.6 shows the comparison of the optimal pH between GoxM10 and the mutants.

### EMBODIMENT

Test materials and reagents:
1. Strains and vectors: *Pichia pastoris* GS115 and expressing vector pPIC9.
2. Enzymes and other biochemical reagents: Endonucleases, high fidelity DNA polymerases and recombinases.
3. LB medium: 0.5% yeast extract, 1% peptone, 1% NaCL, pH 7.0;

*Pichia pastoris* YPD medium:1% yeast extract, 2% peptone, 2% glucose, 7.0; BMGY medium: 1% yeast extract, 2% peptone, 10%YNB, 0.1% Biotin, pH 7.0; BMMY medium: 1% yeast extract, 2% peptone, 10% YNB, 0.1% Biotin, 1.0% methanol (V/V), pH 7.0.

Suitable biology laboratory methods not particularly mentioned in the examples as below can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other kit laboratory manuals.

According to the specific embodiments of the present invention, the glucose oxidase GoxM10 having the amino acid sequence of SEQ ID NO: 1 is subjected to single point mutations of 203, 219, 338, 361, and 419, followed by double point combination mutations of 361/203, 361/219, 361/338, and 361/419/203, 361/419/338, followed by four point combination mutations of 361/419/193/497, 361/419/4/471, and six point combination mutations of 361/419/4/471/193/497, thereby obtaining glucose oxidase mutants with improved acid and heat stability.

Specifically, the glucose oxidase having the amino acid sequence of SEQ ID NO: 1 was mutated as follows:
G203C, E219P, S338P, E361P and A419I;
E361P/G203C, E361P/E219P, E361P/S338P and E361P/A419I;
E361P/A419I/G203C and E361P/A419I/S338;
E361P/A419I/A4D/N471E and E361P/A419I/D193N/D497N;
E361P/A419I/A4D/N471E/D193N/D497N.

### Example 1 Preparing the recombinant strain GS115(pPIC9-goxm10)

The fragment of the gene *goxm10* of 1746bp was amplified by PCR using the primer GoxM10-F(SEQ ID No:30, 30bp)and the primer GoxM10-R(SEQ ID No: 31, 30bp), and the nucleic acid fragment of 8088bp of the vector pPIC9 was obtained by double enzyme digestion, both of which were ligated by recombinase to obtain the recombinant plasmid pPIC9-goxm10 being transformed into the *Escherichia coli* JM109 cells and coated onto LB (containing 100 µ g/mL Amp) for screening. After being sequenced , the recombinant plasmid pPIC9-goxm10 is digested using the restriction endonuclease *BglII*, and the digested product is recovered and transformed into *Pichia pastoris* GS115 by electroporation, to obtain the recombinant expression strain GS115 (pPIC9-goxm10).

### Example 2 Preparation of the recombinant mutant strain GS115

### 1. Constructing the recombinant vector

The mutated amino acids are introduced with a point mutation kit, using the designed mutation primers, and using the plasmid pPIC9-goxm10 as a template, and the PCR products containing mutated amino acids were digested with *Dpn* to remove the template. The digested PCR products are transformed into competent cells of *Escherichia coli* JM109 and sequenced for verifying the mutation, to obtain *E. coli* containing the glucose oxidase mutant plasmid
pPIC9-*goxm10*-G203C,pPIC9-*goxm10*-E219P,pPIC9-*goxm10*-S338P,pPIC9-*gox m10-*E361P,pPIC9-*goxm10-*A419I,pPIC9-*goxm10-*E361P/G203C,pPIC9-*goxm10-*E36 1P/E219P,pPIC9-*goxm10*-E361P/S338P,pPIC9-*goxm10*-E361P/A419I,pPIC9-*goxm10* -E361P/A419I/G203C,pPIC9-*goxm10*-E361P/A419I/S338P,pPIC9-*goxm10*-E361P/A 419I/A4D/N471E, pPIC9-*goxm10*-E361P/A419I/D193N/D497N and pPIC9-*goxm10*-E361P/A419I/A4D/N471E/ D193N/D497N, respectively, wherein

said mutation primers were the primer Gox-G203C-F of SEQ ID No: 32, and the primer Gox-G203C-R of SEQ ID No: 33; the primerGox-E219P-F of SEQ ID No: 34, and the primer Gox-E219P-R of SEQ ID No: 35; the primer Gox-S338P-F of SEQ ID No: 36, and the primer Gox-S338P-R of SEQ ID No: 37; the primer Gox-E361P-F of SEQ ID No: 38, and the primer Gox-E361P-R of SEQ ID No: 39; and, the primer Gox-A419I-F of SEQ ID No:40 and the primer Gox-A419I-R of SEQ ID No: 41.

### 2.Preparing the recombinant strain GS115

The plasmids containing the gene encoding the mutant which have been verified by sequencing, were digested with *Bgl*II, and the digested product was recovered and transformed into competent cells of *Pichia pastoris* GS115 by electroporation, to obtain the recombinant expression strain GS115 (pPIC9-goxm10-G203C),GS115(pPIC9-*goxm10*-E219P),GS115(pPIC9-*goxm10*-S338 P),GS115(pPIC9-*goxm10-*E361P),GS115(pPIC9-*goxm10-*A419I),GS115(pPIC9-*goxm 10-*E361P/G203C),GS115(pPIC9-*goxm10-*E361P/E219P),GS115(pPIC9-*goxm10-*E36 1P/S338P),GS115(pPIC9-*goxm10*-E361P/A419I),GS115(pPIC9-*goxm10*-E361P/A41 9I/G203C),GS115(pPIC9-*goxm10*-E361P/A419I/S338P),GS115(pPIC9-*goxm10*-E361 P/A419I/A4D/N471E), GS115(pPIC9-*goxm10*-E361P/A419I/D193N/D497N) and GS115(pPIC9-*goxm10*-E361P/A419I/A4D/N471E/D193N/D497N).

### Example 3 Detection of the enzymatic properties of mutants

### 1. Inducible expression of GoxM10 and the mutants thereof

The obtained recombinant yeast expression strain were inoculated into 50 mL o f YPD medium with 1% inoculation amount for seed liquid culturing for 48 h ours at 200 rpm and 30°C, followed by being transferred to 400 mL of BMG Y medium with 1% inoculation amount for culturing at 200 rpm and 30°C for 48 hours. And, the bacterial cells were collected by centrifugation and transfer red to 200 mL of BMMY medium for culturing for a total of 72 hours, with a supply of 1% methanol every 24 hours for inducing the expression.

### 2.Purifying GoxM10 and the mutants thereof

The induced bacterial solution was centrifuged at 12000 rpm for 10 minutes, to collect the supernatant and being concentrated with a 10 kDa membrane, dialyzed using 10 mM of disodium hydrogen phosphate citric acid buffer at pH 6.5 , and purified by an anion column with solution A and solution B, wherein the solution A was10 mM of disodium hydrogen phosphate citric acid buffer (pH 6.5) and the solution B is the solution adding 1 M of NaCL to the solution A. The purified protein was obtained by collecting the eluent.

### 3.Detection of the enzymatic properties of GoxM10 and the mutants thereof

### (1) Detecting the thermal stability

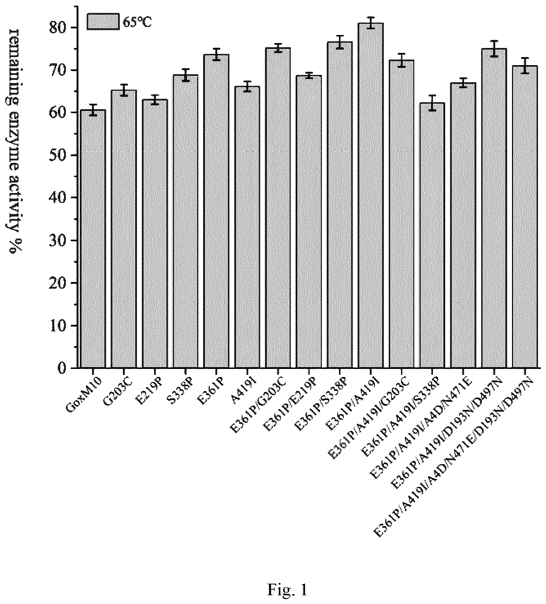

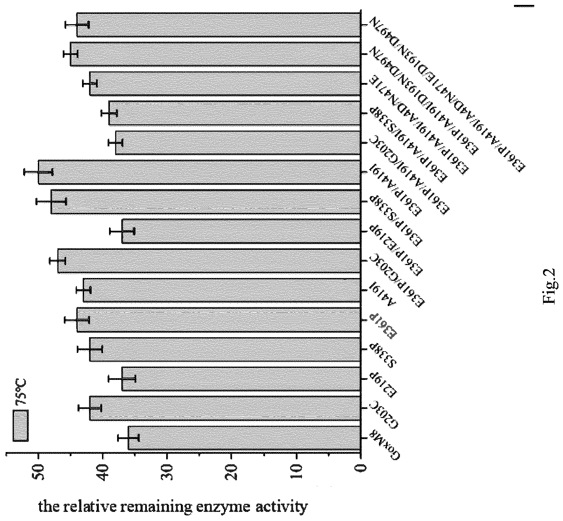

The purified enzyme solution was diluted with 0.1 M of disodium hydrogen phosphate citric acid buffer (pH 6.0) to the same protein concentration, followed by being incubated in water baths at 65°C and 75°C for different time. And, the relative remaining enzyme activity was measured according to standard methods and t_{1/2} was calculated.As shown in Figure 1, after being incubated at 65°C for 180 minutes, the remaining enzyme activity of GoxM10 was 61% while the remaining enzyme activities of mutants G203C, E219P, S338P, E361P, A419I, E361P/G203C, E361P/E219P, E361P/2338P, E361P/A419I/G203C, E361P/A419I/S338P, E361P/A419I/A4D/N471E, E361P/A419I/D193N/D497N, and E361P/A419I/A4D/N471E/D193N/D497N were 65%, 63%, 69%, and 74%, respectively. 66%, 75%, 68%, 76%, 81%, 72%, 62%, 67%, 75%, and 71% being increased by approximately 7%, 3%, 13%, 21%, 8%, 23%, 11%, 25%, 33%, 18%, 2%, 10%, 23%, and 16% respectively. As shown in Figure 2, the remaining enzyme activities of mutants G203C, E219P, S338P, E361P, A419I, E361P/G203C, E361P/E219P, E361P/2338P, E361P/A419I, E361P/A419I/G203C, E361P/A419I/S338P, E361P/A419I/A4D/N471E, E361P/A419I/D193N/D497N, and E361P/A419I/A4D/N471E/D193N/D497N after being incubated at 75°C for 12 minutes were 42%, 37%, 42%, 44%, 43%, 47%, 37%, 48%, 50%, 38%, respectively, being increased by 39%, 42%, 45%, and 44% have increased by approximately 17%, 3%, 17%, 22%, 19%, 31%, 3%, 33%, 38%, 5%, 8%, 17%, 25%, and 22% respectively. As shown in the table 1, the half-life of GoxM10 when being incubated at 65°C is 385 minutes, while the half-lives of mutants E219P and A419I are both 433 minutes, being 1.1 times that of GoxM10. The half-lives of mutants G203C and E361P are 462 minutes and 495 minutes, respectively, being 1.2 and 1.3 times that of GoxM10. The half-lives of mutant S338P are comparable to that of GoxM10. The half-lives of the four double point mutants E361P/G203C, E361P/E219P, E361P/S338P, and E361P/A419I are 495 min, 433 min, 462 min, and 578 min, respectively, which are 1.3, 1.1, 1.2, and 1.5 times that of GoxM10 (385 min). The half-lives of mutants E361P/A419I/G203C, E361P/A419I/S338P, E361P/A419I/A4D/N471E, E361P/A419I/D193N/D497N, and E361P/A419I/A4D/N471E/D193N/D497N are 433, 450, 495, 553, and 462 min, respectively.

**Table 1**

| Enzyme | *t*_{1/2} (min) |
|---|---|
| GoxM8 | 385.08 |
| G203C | 462.10 |
| E219P | 433.22 |
| S338P | 385.08 |
| E361P | 495.11 |
| A419I | 433.22 |
| E361P/G203C | 495.11 |
| E361P/E219P | 433.22 |
| E361P/S338P | 462.10 |
| E361P/A419I | 577.62 |
| E361P/A419I/G203C | 433.22 |
| E361P/A419I/S338P | 450.34 |
| E361P/A419I/A4D/N471E | 495.11 |
| E361P/A419I/D193N/D497N | 553.00 |
| E361P/A419I/A4D/N471E/D193N/D497N | 462.10 |

### (2)Determination of the optimal temperature of GoxM10 and mutants thereof

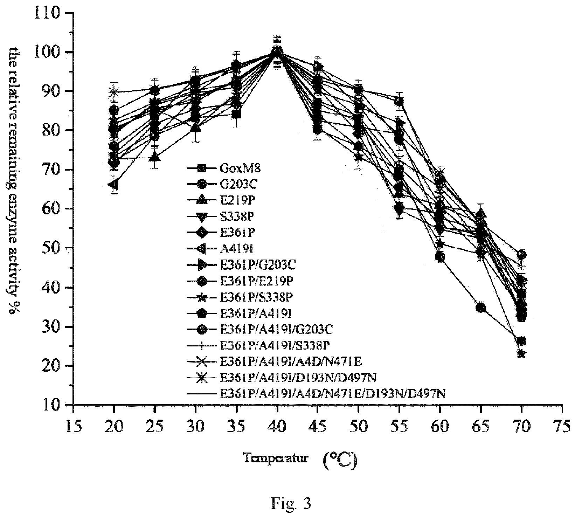

The purified enzyme solution was diluted by an appropriate multiple for measuring the enzyme activity at pH 6.0 and different temperature gradients (20, 25, 30, 35, 40, 45, 50, 55, 60, 65, 70°C) wherein the enzyme activity at the optimal reaction temperature was set as100% , and then calculating the relative enzyme activity at the remaining temperatures to determine the optimal reaction temperature of GoxM10 and its mutants. As shown in Figure 3, the optimal reaction temperature of the mutants G203C, E219P, S338P, E361P, A419I, E361P/G203C, E361P/E219P, E361P/2338P, E361P/A419I, E361P/A419I/G203C, E361P/A419I/S338P, E361P/A419I/A4D/N471E, E361P/A419I/DI93N/D497N and E361P/A419I/A4D/N471E/D193N/D497N is the same as that of GoxM10, which is 40°C.

### (3) Detecting the acid stability

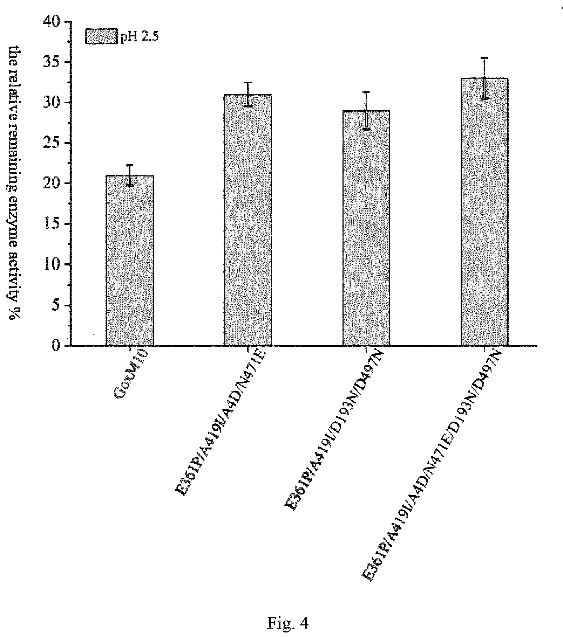

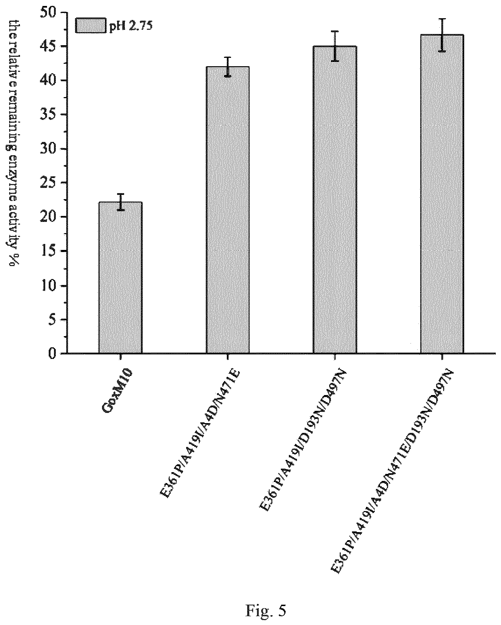

Two measurement methods were used to evaluate the pH stability of GoxM10 and its mutants wherein Method 1 includes the steps of mixing GoxM10 and mutant enzyme solutions of the same concentration with 0.1 M of sodium dihydrogen phosphate citrate buffer solutions at pH 2.5, 2.75, 3.0, 4.0, 5.0, 6.0, 7.0, and 8.0, and then incubating at 37°C for 1 hour to measure the remaining enzyme activity under standard conditions, recording the enzyme activity of the untreated sample as 100%; and Method 2 includes the steps of mixinge GoxM10 and mutant enzyme solutions of the same concentration and 0.1 M of sodium dihydrogen phosphate citric acid buffer at pH 2.5, and then incubating at 37°C for 10 min, 20 min, 30 min, 40 min, 50 min, and 60 min to measure the relative remaining enzyme activity of GoxM10 and its mutant enzyme solution under standard conditions. As shown in Figure 4, after being treated for 30 minutes at pH 2.5, the relative remaining enzyme activities of mutants E361P/A419I/A4D/N471E, E361P/A419I/D193N/D497N, and E361P/A419I/A4D/N471E/D193N/D497N were 31%, 29%, and 33%, respectively, which were increased by about 48%, 38%, and 57% compared to GoxM10 (21%). As shown in Figure 5, after being treated at pH 2.75 and 37°C for 60 minutes, the relative remaining enzyme activities of mutants E361P/A419I/A4D/N471E, E361P/A419I/D193N/D497N, and E361P/A419I/A4D/N471E/D193N/D497N were approximately 42%, 46%, and 47%, respectively, which were increased by about 75%, 92%, and 96% compared to GoxM10 (24%), respectively.

### (4)Determination of optimal pH of GoxM10 and mutants

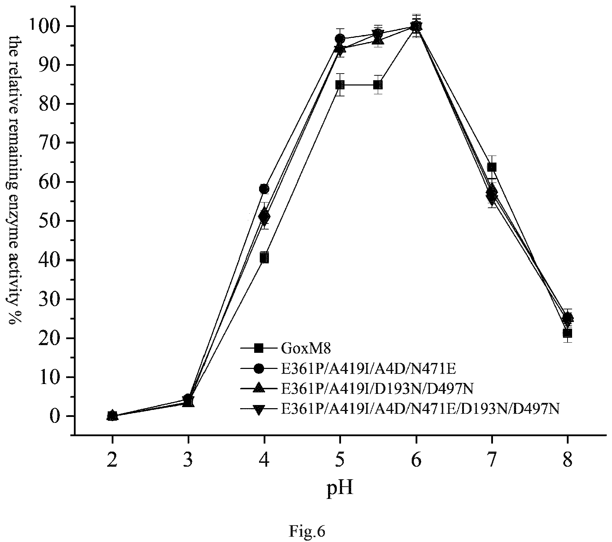

The substrate mixtures were prepared, the enzyme activity of GoxM10 and its mutants at 30°C and different pH gradients of 3.0, 4.0, 5.0, 6.0, 7.0 and 8.0 were measured, and the relative enzyme activity under the different pH conditions were calculated to obtain the optimal reaction pH of GoxM10 and its mutants wherein the enzyme activity at the optimal reaction pH was set as 100%. As shown in Figure 6, the optimal reaction pH for both GoxM10 and its mutants E361P/A419I/A4D/N471E, E361P/A419I/D193N/D497N, and E361P/A419I/A4D/N471E/D193N/D497N is 6.0.

The above embodiments are only used to explain the technical solution of the present application and do not limit the scope of protection of the present application.

## Claims

1. A glucose oxidase mutant with the improved thermal stability wherein the said mutant has an amino acid sequence obtained by performed a mutation of G203C, E219P, S338P, E361P or A419I to the amino acid sequence of SEQ ID NO:1.

2. The glucose oxidase mutant according to claim1, wherein the said amino acid sequence of SEQ ID NO:1 was further performed a mutation of E361P/G203C, E361P/E219P, E361P/S338P or E361P/A419I.

3. The glucose oxidase mutant according to claim 2, wherein the said amino acid sequence of SEQ ID NO:1 was further performed a mutation of E361P/A419I/G203C or E361P/A419I/S338.

4. The glucose oxidase mutant according to claim 2, wherein the said amino acid sequence of SEQ ID NO:1 was further performed a mutation of E361P/A419I/A4D/N471E or E36IP/A419I/D193N/D497N.

5. The glucose oxidase mutant according to claim 3, wherein the said amino acid sequence of SEQ ID NO:1 was further performed a mutation of E361P/A419I/A4D/N471E/D193N/D497N.

6. A gene encoding the glucose oxidase mutant of any one of claims 1 to 5.

7. A method for improving the thermal stability of glucose oxidase wherein the said method includes a step of performing a mutation of G203C, E219P, S338P, E361P or A419I;or E361P/G203C, E361P/E219P, E361P/S338P or E361P/A419I;or E361P/A419I/A4D/N471E or E361P/A419I/D193N/D497N; or E361P/A419I/A4D/N471E/D193N/D497N, to the amino acid sequence of SEQ ID NO:1.

8. A recombinant vector containing the gene of claim 6.

9. A recombinant strain containing the gene of claim 6.

10. An application of the glucose oxidase mutant of any one of claims 1 to 5.
